(19) Europäisches Patentamt
European Patent Office
Office européen des brevets

(11) **EP 2 232 438 B1**

(12) **FASCICULE DE BREVET EUROPEEN**

(45) Date de publication et mention
de la délivrance du brevet:
**04.05.2011 Bulletin 2011/18**

(51) Int Cl.:
***G06T 7/00*** (2006.01)

(21) Numéro de dépôt: **08861890.5**

(22) Date de dépôt: **12.12.2008**

(86) Numéro de dépôt international:
**PCT/EP2008/067444**

(87) Numéro de publication internationale:
**WO 2009/077459 (25.06.2009 Gazette 2009/26)**

(54) **PROCEDE D'ANALYSE D'UNE IMAGE D'HYDRURES DANS UN ALLIAGE METALLIQUE, NOTAMMENT DANS UN ALLIAGE DE GAINAGE DE COMBUSTIBLE NUCLEAIRE**

**VERFAHREN ZUR AUSWERTUNG EINES HYDRIDBILDS IN EINER METALLLEGIERUNG, INSBESONDERE IN EINER KERNBRENNSTOFF-VERKLEIDUNGSLEGIERUNG**

**METHOD OF ANALYZING AN IMAGE OF HYDRIDES IN A METAL ALLOY, IN PARTICULAR IN A NUCLEAR FUEL CLADDING ALLOY**

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **14.12.2007 FR 0708742**

(43) Date de publication de la demande:
**29.09.2010 Bulletin 2010/39**

(73) Titulaire: **Commissariat à l'Énergie Atomique et aux Énergies Alternatives
75015 Paris (FR)**

(72) Inventeurs:
• **ALLEGRE, Stéphane**
  **F-91300 Massy (FR)**
• **SACHOT, Loïc**
  **F-92130 Issy les Moulineaux (FR)**
• **RABOUILLE, Olivier**
  **F-92260 Fontenay aux Roses (FR)**

(74) Mandataire: **Lucas, Laurent Jacques
Marks & Clerk France
Conseils en Propriété Industrielle
Immeuble " Visium "
22, avenue Aristide Briand
94117 Arcueil Cedex (FR)**

(56) Documents cités:
• **J. H. ZHANG, M. GROOS, T. BREDEL, M. TROTABAS, P. COMBETTE: "Quantification et caractérisation des hydrures de zirconium dans le Zircaloy-4 par analyse d'image" JOURNAL OF NUCLEAR MATERIALS, vol. 195, octobre 1992 (1992-10), pages 17-23, XP002486661**
• **RACINE AUDE: "Influence de l'orientation des hydrures sur les modes de déformation, d'endommagement et de rupture du Zircaloy-4 hydruré." THÈSE DE L'ECOLE POLYTECHNIQUE, 23 septembre 2005 (2005-09-23), pages 53-78, XP002518623 palaiseau,framce**
• **F-Y. BRIAND, B. BRILLAULT, S. PHILIPP: "Segmentation de défauts dans des images de radiographies industrielles" REVUE TRAITEMENT DU SIGNAL, vol. 5, no. 4, 1988, pages 291-303, XP002486662**
• **LITOROWICZ ET AL: "Identification and quantification of cracks in concrete by optical fluorescent microscopy" CEMENT AND CONCRETE RESEARCH, PERGAMON PRESS, ELMSFORD, NY, US, vol. 36, no. 8, 1 août 2006 (2006-08-01), pages 1508-1515, XP025011704 ISSN: 0008-8846 [extrait le 2006-08-01]**

**Description**

La présente invention concerne un procédé d'analyse d'une image d'hydrures dans un alliage métallique. Elle s'applique notamment pour l'examen métallographique par analyse d'images, par exemple sur les alliages de Zirconium, matériau de gainage des combustibles utilisés dans les centrales nucléaires.

Un exemple d'une telle analyse est présenté dans l'article de Zhang et al. "Quantification et caractérisation des hydrures de zirconium dans le Zircaloy-4 par analyse d'image", Journal of Nuclear Materials 195, 1992, p.17-23.

Les opérations de contrôles industriels, notamment de contrôle des matériaux, utilisent l'analyse d'images. Ce type d'analyse est notamment appliqué pour le contrôle et la maintenance des gaines des combustibles des centrales nucléaires, ces gaines étant réalisées en Zirconium. A cause de la très faible limite de solubilité de l'hydrogène dans le Zirconium à température ambiante, il précipite avec ce dernier sous forme d'hydrures qui peuvent dans certaines conditions être néfastes à la tenue mécanique des gaines. La quantité d'hydrures formés à la température ambiante est directement proportionnelle à la teneur en hydrogène dans le matériau. L'analyse d'image d'un échantillon de gaine peut être utilisée pour quantifier la teneur en hydrogène présentée par cet échantillon, l'image étant obtenue par exemple par microscopie optique ou par microscopie à balayage électronique. D'autres informations concernant la morphologie des hydrures, par exemple leur taille moyenne, et leur proximité ou leur orientation par rapport à un axe connu sont par ailleurs quantifiables par analyse d'images.

Cette méthode a plusieurs avantages par rapport à la méthode d'extraction à chaud sous vide, en effet c'est une analyse localisée et non destructive pour les hydrures, ce qui est particulièrement important sur les matériaux irradiés. Ainsi il n'est pas nécessaire de détruire les échantillons pour les analyser. Un même échantillon peut alors subir plusieurs examens. Cela permet notamment de rentabiliser l'analyse en évitant d'utiliser trop d'échantillons, dans le cas d'opérations délicates qui demandent beaucoup de manipulations.

Cependant, lors de la préparation des échantillons avant la prise d'images, ces derniers subissent un polissage ainsi qu'une attaque chimique. L'attaque chimique a pour but de révéler les hydrures. Le mélange acide dissout préférentiellement les hydrures qui sont révélés par contraste optique. Malheureusement l'attaque chimique creuse aussi légèrement la matrice autour des hydrures et tend à en accentuer la taille, surtout dans l'épaisseur. Contrairement à un examen par microscopie électronique à balayage, en électrons rétrodiffusés, où l'on peut observer les hydrures dans leur vraie dimension, en microscopie optique un hydrure est d'autant plus large que l'attaque chimique est poussée. Il est donc difficile d'effectuer une analyse fiable, et notamment une caractérisation efficace, des hydrures.

L'invention a notamment pour but de permettre une analyse fiable des hydrures, tant du point de vue quantitatif que qualitatif. A cet effet, l'invention a pour objet un procédé d'analyse d'une image d'hydrures dans un alliage métallique, tel que décrit dans les revendications.

D'autres caractéristiques et avantages de l'invention apparaîtront à l'aide de la description qui suit faite en regard de dessins annexés qui représentent :

- la figure 1, une illustration des étapes possible d'un procédé selon l'invention ;
- les figures 2a à 2d, des illustrations d'une première étape de découpage de l'image d'origine d'un échantillon d'alliage ;
- la figure 3, une illustration d'une étape de binarisation de l'image ;
- la figure 4, une illustration d'une étape de squelettisation de l'image ;
- la figure 5, une illustration d'une étape d'analyse donnant la teneur en hydrogène à l'intérieur de l'échantillon ;
- la figure 6, un exemple de courbe d'étalonnage utilisée pour la détermination de la teneur en hydrogène ;
- la figure 7, des exemples de règles de connexité pour définir l'appartenance de pixels à un même hydrure ;
- la figure 8, une illustration de la proximité entre deux hydrures.

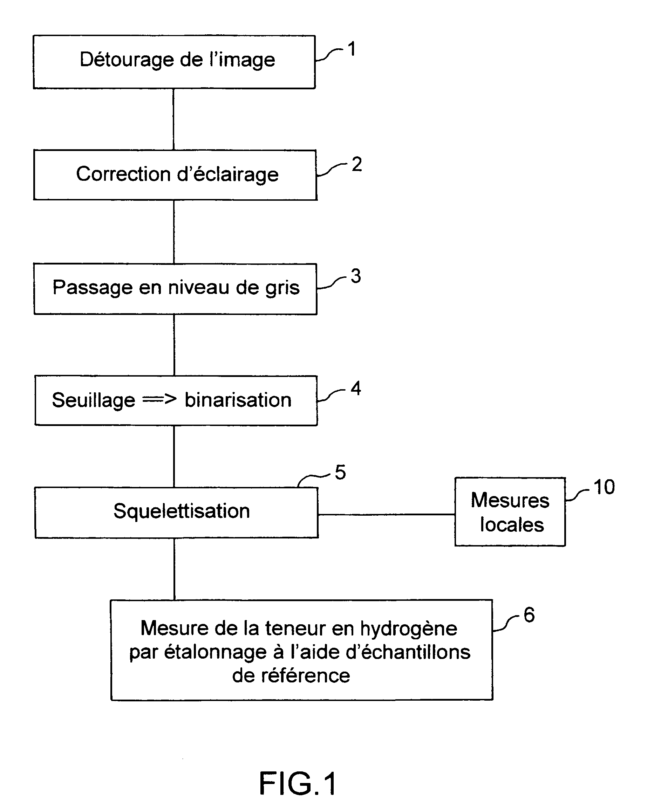

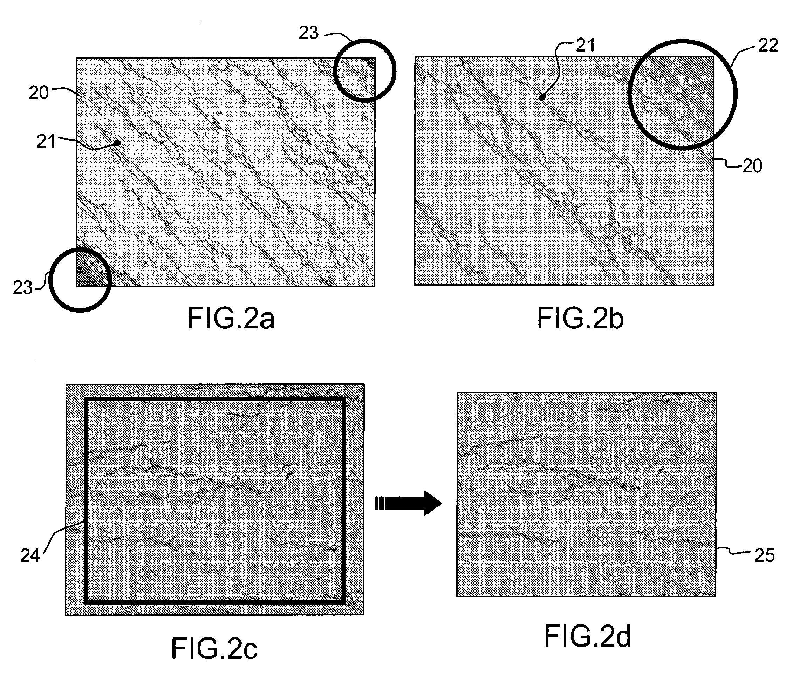

La figure 1 illustre les étapes possibles d'un procédé selon l'invention, appliqué par exemple à l'analyse des hydrures présents dans une gaine en alliage de Zirconium des crayons combustibles après irradiation dans une centrale nucléaire. Dans une étape préliminaire, on réalise une image d'un échantillon d'une gaine. Cette image est prise par microscopie optique ou par tout autre moyen. La figure 2a présente un exemple d'image obtenue, comportant par exemple 760 x 570 pixels. Cette image 20 montre les traces d'hydrures 21 présents dans l'échantillon.

Dans une première étape 1, l'image est détourée, puis dans des étapes suivantes 2, 3, 4, 5 elle est traitée jusqu'à obtenir une image de type squelettique, c'est-à-dire où les traces d'hydrures sont réduites à l'épaisseur d'un pixel. Enfin dans une dernière étape 6, la teneur en hydrogène est déterminée sur la base de l'image squelettisée à l'aide d'échantillons de référence par une méthode d'étalonnage.

Les figures 2a à 2d illustrent la première étape 1 de détourage de l'image 20 en vue d'obtenir notamment une image de dimensions paramétrables, centrée sur l'image d'origine. Cette opération permet en particulier d'éliminer les principaux problèmes liés aux effets de bords tels que par exemple :

- les zones de flou 22 sur les bords, relatifs à la prise de l'image ;
- la présence de pixels hors gaine 23 au bord de l'image.

La figure 2c illustre le détourage 24 effectué donnant l'image détourée 25 de la figure 2d. C'est cette image 25 qui est traitée par la suite.

La première étape 1 peut être suivie d'une étape 2 de correction d'éclairage de l'image détourée 25. Lors de l'acquisition d'images, l'opérateur règle lui-même l'intensité de l'éclairage. Les lampes utilisées ont généralement des performances variables dans le temps qui rendent également l'éclairage variable. Les échantillons peuvent aussi avoir un pouvoir de réflectance différent. Cette deuxième étape 2 permet alors si nécessaire de réduire les artéfacts d'éclairage.

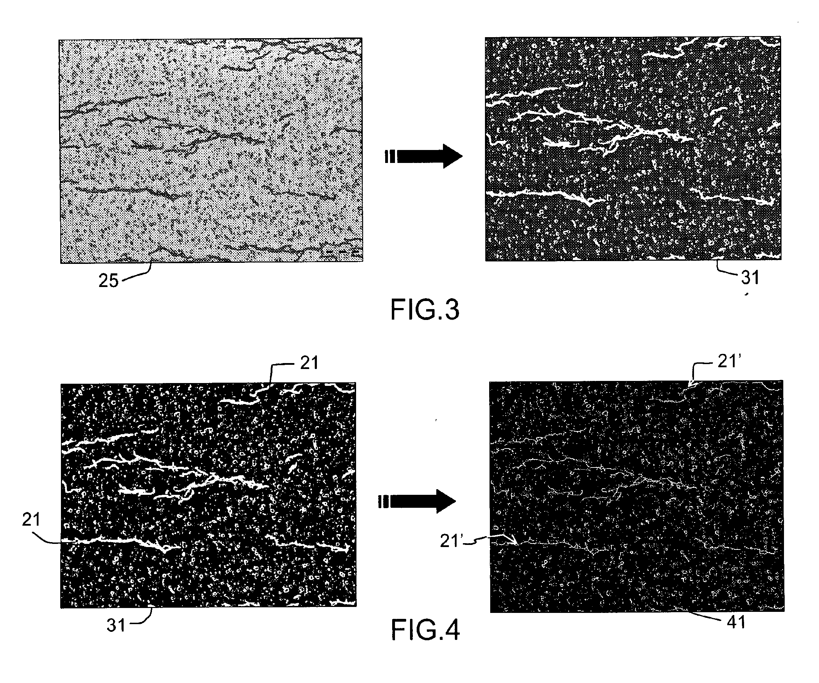

L'étape suivante 3 effectue un passage en niveaux de gris de l'image colorisée 25. Le passage en niveaux de gris permet de ne pas conserver une image en couleur qui peut être trop complexe pour effectuer le seuillage de l'étape suivante 4. Plusieurs solutions connues sont possibles pour réaliser un passage en niveaux de gris. L'image 25 de la figure 3 est par exemple obtenue selon le passage par intensité, le passage en niveaux de gris se faisant en fonction de l'intensité lumineuse. D'autres solutions restent cependant possibles.

La figure 3 illustre l'étape suivante 4 qui effectue un seuillage suivi d'une binarisation de l'image. Plusieurs méthodes de seuillages sont connues. Le but du seuillage est de sélectionner les hydrures en ne prenant que le minimum d'éléments non souhaités. Le seuillage peut être nécessaire pour l'analyse d'une image. En effet de cette opération dépend la binarisation de l'image.

La binarisation de l'image suit l'opération de seuillage. La binarisation consiste au passage de l'image en niveau de gris 25 en une image en noir et blanc 31. Cette opération permet de simplifier encore plus le traitement d'image en vue d'une sélection de particules à l'intérieur de cette image.

La figure 4 illustre le résultat obtenu lors de l'étape 5 suivante, dite de squelettisation. Cette opération est un traitement d'érosion ultime d'un objet à la taille d'un pixel d'épaisseur. La figure 4 présente l'image 31 avant cette opération de squelettisation et l'image 41 obtenue. Les hydrures 21 de l'image 31 sont alors transformés en des filaments 21' d'une épaisseur d'un pixel. Le mode de squelettisation utilisé dans l'exemple de la figure 4 est du type « squelettisation claire ». Dans ce cas, on ne réalise la squelettisation que des pixels blancs sur fond noir, les pixels blancs correspondants aux hydrures. Il est possible d'utiliser plusieurs degrés de squelettisation. Le degré maximum transforme les hydrures en des filaments d'un pixel d'épaisseur. Des degrés de squelettisation inférieurs peuvent être utilisés, les hydrures sont alors transformés en des filaments d'épaisseurs supérieures à un pixel, l'épaisseur finale dépendant du degré.

L'invention utilise avantageusement le fait que les hydrures ont, pour un matériau donné, sensiblement la même épaisseur et cela quelle que soit leur concentration dans l'échantillon. L'invention utilise par ailleurs comme hypothèse que la teneur en hydrogène d'un alliage de Zirconium est directement proportionnelle à la longueur cumulée des hydrures. Cela permet alors d'assimiler un hydrure à un filament sans considérer son épaisseur. L'étape de squelettisation 5 permet d'obtenir les filaments à partir desquels les mesures de teneur vont être effectuées dans l'étape suivante 6.

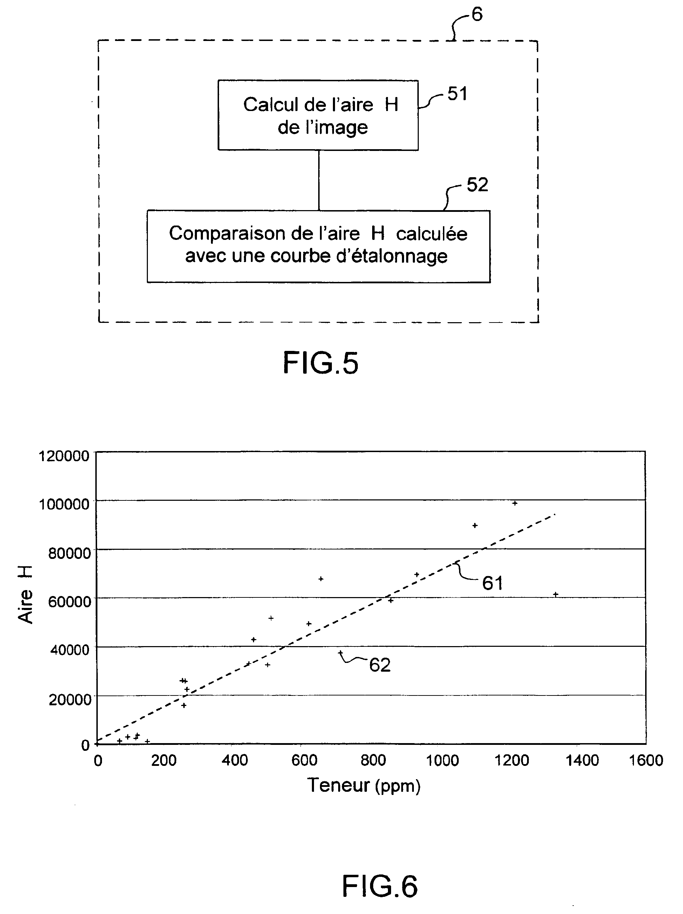

La figure 5 présente les phases possibles de cette étape 6 de mesure de la teneur en hydrogène. Dans une première phase 51 une grandeur caractéristique de cette teneur est calculée, appelée aire H par la suite. Cette grandeur prend en compte les aires des filaments correspondant à des hydrures.

Plus particulièrement, c'est une grandeur sans dimension correspondant au rapport de l'aire totale des filaments d'hydrure détectés sur l'aire totale de l'image squelettisée 41. Elle est alors définie par la relation suivante :

$$Aire\ H = \frac{\sum Aires\ des\ filaments\ d'hydrures\ détectés}{Aire\ totale\ de\ l'image} \qquad (1)$$

En pratique, elle peut être définie par la relation suivante :

$$Aire\ H = \frac{\sum Aire\ des\ pixels\ blancs\ retenus}{Aire\ totale\ de\ l'image} \qquad (2)$$

Les pixels blancs retenus, correspondant à des filaments d'hydrures détectés 21', dépendent d'options de mesures qui seront définis par la suite. Chaque aire de la somme E est celle d'un groupement de pixels formant une particule. Les options de mesures définissent des règles pour ne retenir que les particules correspondant à des filaments d'hydrures.

Dans une deuxième phase 52 l'aire H calculée à partir des filaments d'hydrures détectés, selon la relation (2) par exemple, est comparée avec une courbe d'étalonnage pour obtenir la teneur en hydrogène à l'intérieur de l'échantillon.

La figure 6 donne un exemple de courbe d'étalonnage 61. L'axe des ordonnées représente les valeurs de l'aire H et l'axe des abscisses représente la teneur en hydrogène, exprimée en ppm.

Cette courbe d'étalonnage est réalisée avec les mêmes options de mesures que celles citées ci-dessus pour la sélection des filaments. Cette courbe d'étalonnage 61 est définie à partir de mesures effectuées sur des échantillons de référence présentant une répartition homogène des hydrures et dont on connaît la teneur en hydrogène. Pour chacun de ces échantillons on calcule l'aire H, selon la relation (2) par exemple, selon le même mode de calcul que pour l'image 41 de l'échantillon à analyser. Pour des raisons de lisibilité, l'aire H calculée selon la relation (2) est par exemple multipliée par $10^6$ de sorte que sa valeur varie entre 0 et 120000 sur l'axe des ordonnées. Les mesures effectuées sur les échantillons de référence sont représentées par des croix 62 dans le système d'axes de la figure 6. Pour chaque échantillon de référence, on connaît a priori la teneur en hydrogène repérée sur l'axe des abscisses, on calcule son aire H que l'on reporte sur l'axe des ordonnées pour obtenir le point de mesure correspondant 62.

La courbe d'étalonnage 61 est alors par exemple une droite dont la position est définie à partir de la position des points de mesure 62 des échantillons de référence. La droite 61 est par exemple la droite qui présente la distance minimum par rapport à l'ensemble des points de mesures 62. Une fois cette courbe d'étalonnage établie, la teneur en hydrogène contenue dans l'image 41 de l'échantillon à analyser est déterminée à partir du calcul de son aire H. A partir de l'aire H calculée reportée sur l'axe des ordonnées, on détermine à l'aide de la courbe 61 la valeur de l'axe des abscisses correspondante qui correspond à la teneur en hydrogène recherchée. La méthode est ici décrite de façon manuelle par report d'une valeur calculée sur un axe des ordonnées. La courbe 61 peut bien sûr être mise en équation et la teneur obtenue automatiquement par calcul à partir de l'équation de la courbe 61 et de la valeur de l'aire H de l'échantillon à analyser.

Ce procédé de mesure de la teneur en hydrogène permet avantageusement de traiter en globalité un grand nombre d'images et répond parfaitement aux besoins de quantification des hydrures dans les matériaux.

Pour assurer une bonne fiabilité de la méthode, il est nécessaire de prévoir un nombre d'échantillons de référence, et donc de points de mesure 62, suffisant. Toute modification des options de mesures nécessite par ailleurs un nouvel échantillonnage, c'est-à-dire la définition d'une nouvelle courbe d'étalonnage 61.

Plusieurs options de mesures peuvent être appliquées pour la détection des particules, c'est-à-dire des groupements de pixels. On peut notamment retenir les trois options relatives aux paramètres suivant :

- la connexité des pixels pour déterminer l'appartenance de pixels à une même particule ;
- le nombre minimum de pixels dans une particule pour statuer s'il s'agit ou non d'un hydrure ;
- les inclusions sur les bords pour déterminer si les particules qui ont un ou plusieurs pixels sur les limites de l'image 41 sont inclues ou non dans les résultats de mesures.

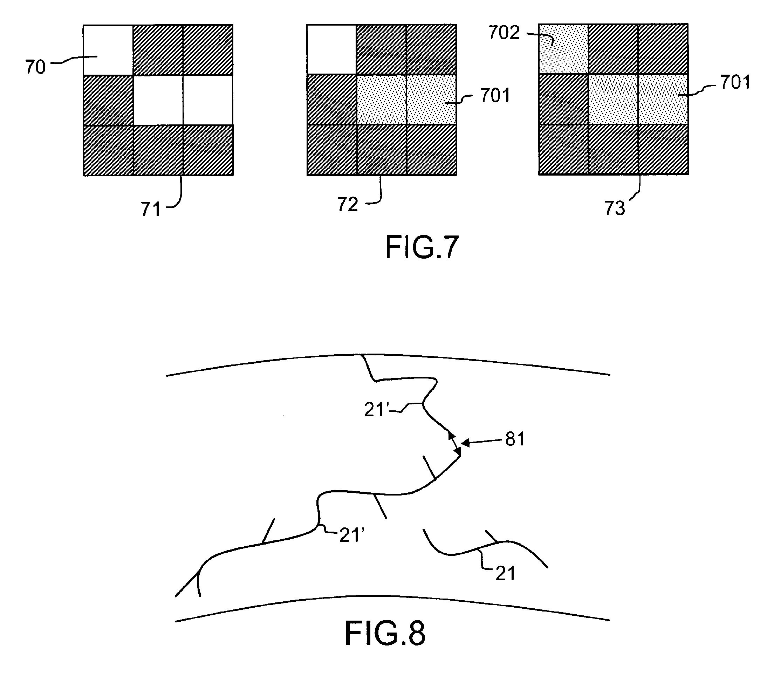

En ce qui concerne la connexité, c'est-à-dire le nombre de voisins à prendre en compte, c'est un paramètre qui permet d'élargir les particules. La figure 7 illustre deux modes de sélection possibles de pixels voisins à partir d'un extrait d'image carré 71 d'origine comportant 3x3 = 9 pixels dont 3 pixels blancs 70.

Le carré 72 illustre l'option de sélection à 4 voisins. Dans cette option, on considère que deux pixels de même couleur 701 vont faire partie d'une même particule uniquement s'ils sont situés sur des pixels voisins directement, c'est-à-dire soit au-dessus, en dessous, à droite ou à gauche, comme l'illustrent les deux pixels blancs d'origine 701 qui ont été grisés sur le carré 72.

Le carré 73 illustre l'option de sélection à 8 voisins. Dans cette option, on élargit le champ de détection, c'est-à-dire que dorénavant les pixels voisins en diagonal 702 sont considérés comme faisant partie de la même particule. Les pixels blancs d'origine du carré 70, grisés 701, 702 sur le carré 73 font ainsi partie d'une même particule.

Une fois déterminée la règle d'appartenance de pixels à une même particule, il est nécessaire de définir le nombre minimum de pixels que doit comporter une particule pour correspondre à un hydrure. On peut par exemple prendre ce nombre minimum égal à 10 et ainsi éliminer toutes les particules inférieures à 10 pixels. Ce filtrage permet notamment d'éliminer la majorité des piqûres dues à la préparation des échantillons. En effet, grâce à la squelettisation les piqûres ont été réduites à des particules unitaires ou de petite taille. Lorsqu'on réalise la squelettisation d'une forme allongée, comme un hydrure par exemple, on obtient un filament d'épaisseur égale à un pixel. Si on réalise la même opération sur une forme arrondie comme une piqûre par exemple, on obtient soit une forme arrondie courte d'épaisseur égale à un pixel, soit un seul pixel isolé. Ainsi par la squelettisation, suivie de ce filtrage, on peut éliminer les défauts dus aux piqûres et aux poussières par exemple.

Enfin, les options relatives aux inclusions sur les bords permettent de déterminer si les particules ayant un ou plusieurs pixels sur les bords doivent être pris en compte pour les mesures. Deux options sont possibles. Dans une première option les particules touchant les bords sont inclues dans les mesures. Cette option est notamment utile pour le calcul de la teneur d'hydrure dans une image. Dans une deuxième option, les particules touchant les bords sont exclues des mesures. Cette option peut être choisie dans le cas de la détermination de la longueur moyenne des hydrures par exemple.

La mesure de la teneur en hydrogène à l'intérieur d'un échantillon, valeur brute quantitative sans dimension, peut être avantageusement complétée par des mesures qualitatives locales des hydrures dans leur contexte. Ces mesures sont toujours réalisées à partir de l'image squelettisée 41. En effet, le danger impliqué par les hydrures dépend notamment de leurs morphologies, incluant aussi leur proximité ainsi que leurs orientations, particulièrement en ce qui concerne la propagation éventuelle d'une fissure.

On revient à la figure 1 qui montre qu'en fin d'étape de squelettisation 5, il est possible de réaliser par ailleurs, dans une autre étape d'analyse 10, des mesures locales, ces mesures étant par exemple les mesures qualitatives précitées, c'est-à-dire la longueur des hydrures, l'orientation des hydrures et leur proximité correspondant à la distance entre plus proches voisins.

L'indice de longueur d'un hydrure est important lorsqu'il s'agit de caractériser son danger pour le matériau. En effet, plus un hydrure est long, plus une fissure peut parcourir un long chemin dans cet hydrure à condition que son orientation soit favorable vis-à-vis de la contrainte appliquée au matériau. Dans le gainage du combustible l'orientation radiale des hydrures est la plus critique en ce qui concerne la fragilité alors que l'orientation circonférentielle est favorable au bon comportement du matériau. Plusieurs critères de longueur peuvent ainsi être définis. Il est bien sûr possible de rechercher la longueur la plus importante de l'hydrure s'il s'agit par exemple d'hydrures radiaux. Mais dans le cas d'hydrures mixtes ou majoritairement circonférentiels il peut être plus utile de définir la longueur maximum qui peut être parcourue dans la direction radiale par un hydrure.

Plusieurs solutions sont possibles pour déterminer la longueur. Dans une solution simple à mettre en oeuvre, pour chaque particule, on prend les points le plus haut et le plus bas selon l'axe vertical de la photographie correspondant à l'axe radial. Puis on fait la différence entre les deux ordonnées qui donne la longueur radiale des hydrures. Cette longueur est importante à considérer car elle permet de caractériser, en cas de propagation de la fissure, la distance qu'elle peut parcourir facilement dans le sens radial de la gaine. Dans le cas d'un hydrure orienté radialement, cette distance peut être grande. Dans le cas d'un hydrure circonférentiel, cette distance est nulle ou très faible. Une autre mesure de la longueur d'un hydrure consiste à trouver les deux points les plus éloignés dans l'hydrure et à mesurer la distance les séparant, cette distance étant alors considérée comme la longueur de l'hydrure. Enfin, il est toujours possible de définir la longueur d'un hydrure comme la somme des longueurs de ses morcellements ou branches.

L'indice d'orientation est aussi un paramètre important dans l'analyse locale pour caractériser le danger des hydrures. En effet, si on admet que l'hydrure est plus fragile que la matrice, une orientation radiale des hydrures est alors favorable à la propagation d'une fissure pouvant conduire à une rupture de la gaine. Il est donc important de caractériser l'orientation des hydrures pour pouvoir déterminer leur danger. Dans une image squelettisée 41 du type de celle de la figure 4, un hydrure est un filament très ramifié et l'orientation moyenne d'un hydrure n'est pas toujours le paramètre le plus judicieux pour caractériser son danger. Il est donc nécessaire de développer différents paramètres de mesure d'orientation représentatifs d'un risque et adapté à la morphologie particulière des hydrures.

Plusieurs définitions de l'orientation d'un hydrure peuvent être utilisées. Dans une première solution, on définit une orientation moyenne $\theta_{moy}$ selon la relation suivante :

$$\theta_{moy} = \frac{\sum_i \theta_i d_i}{\sum_i d_i} \qquad (3)$$

où i représente le rang d'un morcellement de l'hydrure, $\theta_i$ représente l'orientation du morcellement de rang i par rapport à un axe de référence et $d_i$ représente la longueur du morcellement de rang i.

On obtient ainsi une valeur moyenne de l'orientation qui est en rapport avec les longueurs des morcellements associés. Cette définition de l'orientation peut être utile dans le cas où l'on souhaite voir l'éparpillement de la valeur d'orientation à cause des différentes ramifications.

Dans une autre définition de l'angle d'orientation, on prend les deux points les plus éloignés de chaque particule. Puis on calcule l'orientation entre ces deux points qui est prise comme orientation de la particule. Dans une autre définition, au lieu de considérer les deux points les plus éloignés on prend l'orientation entre les deux intersections les plus éloignées.

L'indice de proximité représente le rapprochement entre les hydrures dans un certain sens de propagation. Cela signifie que, si on considère un hydrure A, on caractérise le fait qu'en cas de propagation de fissure, cette dernière pourra se propager facilement ou non vers un hydrure B lorsqu'elle atteindra une extrémité de l'hydrure A. Plus la distance entre les extrémités de l'hydrure A et celles de l'hydrure B est petite, plus il est facile pour une fissure de passer de l'un à l'autre. La disposition des hydrures doit être prise en compte car deux hydrures qui sont voisins, parallèles, radiaux et de mêmes longueurs ne sont pas des cas fiables pour caractériser la proximité. Le cas de deux hydrures dans le prolongement l'un de l'autre est un cas critique dans un contexte de proximité. Plusieurs approches sont cependant possibles. On peut considérer que les fissures se propagent dans un matériau non hydruré de façon purement radiale.

On peut aussi considérer que les fissures se propagent à $\pm$ 45° par rapport à l'axe radial, phénomène de cisaillement. Une troisième approche propose que la fissure poursuit son cheminement dans la matrice selon l'orientation de la dernière portion d'hydrure qu'elle a traversé.

Plusieurs solutions sont possibles pour définir la proximité entre deux particules. La figure 8 illustre un exemple où la proximité est définie par la distance 81 entre les extrémités des hydrures, représentés par les filaments 21', les plus proches voisins. Pour déterminer cette distance, on calcule par exemple la distance entre chaque extrémité d'un hydrure 21' et les extrémités des autres hydrures 21', selon un angle compris entre 0° et 180°, c'est-à-dire dans la continuité de l'hydrure testé. Pour chaque extrémité d'un hydrure, on prend le minimum de cette distance, si le critère d'orientation a été validé. La valeur de proximité p est le minimum de ces distances minimum. Une autre méthode, bidirectionnelle, est possible. Le processus est le même que précédemment sauf que l'on exécute un double passage, d'une part entre 0° et 180° et d'autre part entre 180° et 360°. On obtient ainsi deux distances. La valeur de proximité retenue peut être la moyenne de ces deux distances. Au lieu de prendre la moyenne des deux distances, il est aussi possible de prendre le minimum de ces deux distances.

Selon l'invention on définit pour chaque hydrure, représenté par un filament 21', une grandeur D qui caractérise le danger localement, dans l'environnement plus global d'une gaine de combustible par exemple. Ce facteur de danger D prend en compte la longueur L de l'hydrure, son angle d'orientation θ et sa proximité p à un autre hydrure. Ces paramètres L, θ et p sont par exemple définis selon les méthodes précédemment décrites. La grandeur D est définie par exemple par la relation suivante :

$$D = \sqrt{L^2 + \theta^2 + \frac{1}{p^2}} \qquad (4)$$

L et p sont par exemple définis en $\mu$m et θ en degrés.

Un facteur de danger moyen $D_{moy}$ peut être défini pour l'ensemble des hydrures de l'image, ce facteur de danger étant par exemple la moyenne des facteurs de danger de tous les hydrures. Il est aussi possible de définir un facteur de danger maximum $D_{max}$ correspondant au facteur de danger maximum sur l'ensemble des hydrures de l'image.

Le facteur de danger maximum permet avantageusement de connaître pour chaque image l'hydrure le plus dangereux. Le facteur de danger moyen des images permet de comparer ces images entre elles, donc les matériaux entre eux.

L'invention a été décrite pour l'application à un alliage de Zirconium, elle peut être appliquée pour l'analyse d'hydrures dans d'autres types d'alliage.

## Revendications

1. Procédé d'analyse d'une image d'hydrures dans un alliage métallique, l'image d'origine (20) composée de pixels (70) représentant un échantillon de l'alliage, les hydrures étant représentés par des groupements de pixels (21 ), ledit procédé comportant des étapes de traitement de l'image (1, 2, 3, 4, 5) pour obtenir le squelette des groupements de pixels (21') contenus dans l'image, l'étape de squelettisation (5) étant suivie d'une étape (6, 10) d'analyse portant sur les groupements ainsi squelettisés, l'étape d'analyse (6) exécute un calcul de la teneur en hydrogène à l'intérieur de l'échantillon, **caractérisé en ce qu'**une étape d'analyse (10) délivre un facteur de danger d'un hydrure (21), ce facteur de danger étant une valeur fonction de la morphologie de l'hydrure dans sa représentation squelittisée (21'), le facteur de danger d'un hydrure étant une grandeur D définie selon la relation suivante :

$$D = \sqrt{L^2 + \theta^2 + \frac{1}{p^2}}$$

où L représente la longueur de l'hydrure squelettisé (21'), θ son orientation et p sa proximité avec les autres hydrures.

2. Procédé selon la revendication 1, **caractérisé en ce que** la teneur est déterminée par le calcul d'une aire H composée de l'ensemble des groupements de pixels (21') de l'image représentatifs des hydrures, ladite aire H étant comparée à une courbe d'étalonnage (61) construite à partir d'échantillons de référence dont la teneur en hydrogène est connue, un groupement de pixels représentatif d'un hydrure répondant à des options de mesures prédéterminées, la courbe d'étalonnage étant une courbe représentative de l'aire H en fonction de la teneur en hydrogène, la courbe d'étalonnage étant définie à partir de points de mesures correspondants aux couples formés de l'aire H et de la teneur en hydrogène correspondante des échantillons de référence, le calcul de l'aire H étant effectué en respectant les mêmes options de mesures que pour l'échantillon à analyser.

3. Procédé selon l'une quelconque des revendications précédentes, **caractérisé en ce que** l'alliage métallique est un alliage de Zirconium.

4. Procédé selon l'une quelconque des revendications précédentes, **caractérisé en ce que** l'alliage métallique forme une gaine de protection des crayons combustible nucléaire.

5. Procédé selon l'une quelconque des revendications précédentes, **caractérisé en ce que** l'aire H est égale à la somme des aires des groupements de pixels (21') représentatifs des hydrures sur l'aire totale de l'image (41).

6. Procédé selon l'une quelconque des revendications précédentes, **caractérisé en ce que** la courbe d'étalonnage (61) présente la distance minimum à partir de l'ensemble des points de mesures des échantillons de référence.

7. Procédé selon l'une quelconque des revendications précédentes, **caractérisé en ce que** la longueur L de l'hydrure est définie par la somme des longueurs des branches de sa représentation squelettisée (21').

8. Procédé selon l'une quelconque des revendications 1 à 6, **caractérisé en ce que** la longueur L de l'hydrure est définie par la distance entre les deux points les plus éloignés de sa représentation squelettisée (21') parallèlement à un axe.

9. Procédé selon l'une quelconque des revendications 1 à 6, **caractérisé en ce que** la longueur L de l'hydrure est définie par la distance entre les deux points les plus éloignés de sa représentation squelettisée (21').

10. Procédé selon l'une quelconque des revendications précédentes, **caractérisé en ce que** l'orientation est définie par une orientation moyenne $\theta_{moy}$ selon la relation suivante :

$$\theta_{moy} = \frac{\sum_i \theta_i d_i}{\sum_i d_i}$$

où i représente le rang de la branche de l'hydrure, $\theta_i$ représente l'orientation du morcellement de rang i par rapport à un axe de référence et $d_i$ représente la longueur du morcellement de rang i.

11. Procédé selon l'une quelconque des revendications 1 à 9, **caractérisé en ce que** l'orientation est l'orientation entre les deux points les plus éloignés de l'hydrure.

12. Procédé selon l'une quelconque des revendications 1 à 9, **caractérisé en ce que** l'orientation est l'orientation entre les deux intersections de branches les plus éloignées de l'hydrure.

13. Procédé selon l'une quelconque des revendications précédentes, **caractérisé en ce que** la proximité p étant définie par la distance (81) entre la proximité des hydrures les plus proches voisins, cette distance étant définie par le calcul de la distance entre chaque extrémité d'un hydrure (21') et les extrémités des autres hydrures (21') selon un angle compris entre 0° et 180°, pour chaque extrémité on prend le minimum de cette distance, la valeur de la proximité étant le minimum des distances minimum.

14. Procédé selon la revendication 13, **caractérisé en ce que** le calcul de la distance entre chaque extrémité d'un hydrure (21') et les extrémités des autres hydrures (21') selon un angle compris entre 0° et 180° d'une part, et entre 180° et 360° d'autre part.

15. Procédé selon l'une quelconque des revendications précédentes, **caractérisé en ce qu'**un facteur de danger $D_{moy}$ est défini pour l'échantillon, ce facteur de danger $D_{moy}$ étant la moyenne des facteurs de danger des hydrures présents sur l'image (41) de l'échantillon.

16. Procédé selon l'une quelconque des revendications précédentes, **caractérisé en ce qu'**un facteur de danger maximum $D_{max}$ est défini pour l'échantillon, ce facteur de danger $D_{max}$ correspondant au facteur de danger maximum sur l'ensemble des hydrures présents sur l'image (41) de l'échantillon.

**Claims**

1. A process for analysing an image of hydrides in a metal alloy, the original image (20) being composed of pixels (70) representing a sample of the alloy, the hydrides being represented by groups of pixels (21), said process comprising steps of processing the image (1, 2, 3, 4, 5) to obtain the skeleton of the groups of pixels (21') contained in the image, the skeletonisation step (5) being followed by a step (6, 10) of analysing the groups thus skeletonised, the analysis step (6) including a calculation of the hydrogen content inside the sample, **characterised in that** an analysis step (10) provides a danger factor for a hydride (21), this danger factor being a value which is a function of the morphology of the hydride in its skeletonised representation (21'), the danger factor of a hydride being a value D defined according to the following relation:

$$D = \sqrt{L^2 + \theta^2 + \frac{1}{p^2}}$$

where L represents the length of the skeletonised hydride (21'), θ its orientation and p its proximity with the other hydrides.

2. The process according to claim 1, **characterised in that** the content is determined by the calculation of an area H composed of all of the groups of pixels (21') of the image representative of the hydrides, said area H being compared with a calibration curve (61) created from reference samples with known hydrogen content, a group of pixels representative of a hydride fulfilling the predetermined measurement options, the calibration curve being a curve which represents the area H according to the hydrogen content, the calibration curve being defined from measuring points corresponding to the pairs formed in the area H and the corresponding hydrogen content of the reference samples, the calculation of the area H being performed in accordance with the same measuring options as for the sample to be analysed.

3. The process according to any one of the preceding claims, **characterised in that** the metal alloy is a zirconium alloy.

4. The process according to any one of the preceding claims, **characterised in that** the metal alloy forms a protective cladding for nuclear fuel rods.

5. The process according to any one of the preceding claims, **characterised in that** the area H is equal to the sum of the areas of the groups of pixels (21') which represent hydrides over the total area of the image (41).

6. The process according to any one of the preceding claims, **characterised in that** the calibration curve (61) shows the minimum distance from all of the measuring points of the reference samples.

7. The process according to any one of the preceding claims, **characterised in that** the length L of the hydride is defined by the sum of the lengths of the branches in its skeletonised representation (21').

8. The process according to any one of claims 1 to 6, **characterised in that** the length L of the hydride is defined by the distance between the two farthest points of its skeletonised representation (21') parallel to an axis.

9. The process according to any one of claims 1 to 6, **characterised in that** the length L of the hydride is defined by the distance between the two farthest points of its skeletonised representation (21').

10. The process according to any one of the preceding claims, **characterised in that** the orientation is defined by an average orientation θmoy according to the following relation:

$$\theta_{moy} = \frac{\sum_i \theta_i d_i}{\sum_i d_i}$$

where i represents the row of the hydride branch, θ1 represents the orientation of the division of the row i relative to a reference axis and di represents the length of the division of row i.

11. The process according to any one of claims 1 to 9, **characterised in that** the orientation is the orientation between the two farthest points of the hydride.

12. The process according to any one of claims 1 to 9, **characterised in that** the orientation is the orientation between the two intersections of branches farthest from the hydride.

13. The process according to any one of the preceding claims, **characterised in that** as the proximity p is defined by the distance (81) between the proximity of the closest neighbouring hydrides, this distance being defined by the calculation of the distance between each end of a hydride (21') and the ends of the other hydrides (21') over an angle of between 00 and 1800, for each end the minimum of this distance is selected, the value of the proximity being the minimum of the minimum distances.

14. The process according to claim 13, **characterised in that** the calculation of the distance between each end of a hydride (21') and the ends of the other hydrides (21') is performed over an angle of between 00 and 1800 on the one hand and between 1800 and 3600 on the other hand.

15. The process according to any one of the preceding claims, **characterised in that** a danger factor Dmoy is defined for the sample, this danger factor Dmoy being the average of the danger factors of the hydrides present on the image (41) of the sample.

16. The process according to any one of the preceding claims, **characterised in that** a maximum danger factor Dmax is defined for the sample, this danger factor Dmax corresponding to the maximum danger factor for all of the hydrides present on the image (41) of the sample.

**Patentansprüche**

1. Verfahren zum Analysieren eines Bildes von Hydriden in einer Metalllegierung, wobei das Originalbild (20) aus Pixeln (70) zusammengesetzt ist, die für eine Probe der Legierung repräsentativ sind, wobei die Hydride von Pixelgruppen (21) repräsentiert werden, wobei das Verfahren die Schritte des Verarbeitens des Bildes (1, 2, 3, 4, 5) zum Erhalten des Skeletts der in dem Bild enthaltenen Pixelgruppen (21') beinhaltet, wobei auf den Skelettierschritt (5) ein Schritt (6, 10) des Analysierens der so skelettierten Gruppen folgt, wobei der Analyseschritt (6) eine Berechnung des Wasserstoffgehalts innerhalb der Probe beinhaltet, **dadurch gekennzeichnet, dass** ein Analyseschritt (10) einen Gefahrenfaktor für ein Hydrid (21) ergibt, wobei dieser Gefahrenfaktor ein Wert ist, der von der Morphologie des Hydrids in seiner skelettierten Darstellung (21') abhängig ist, wobei der Gefahrenfaktor eines Hydrids ein Wert D ist, der durch die folgende Relation definiert wird:

$$D = \sqrt{L^2 + \theta^2 + \frac{1}{p^2}}$$

wobei L die Länge des skelettierten Hydrids (21'), θ seine Orientierung und p seine Nähe zu den anderen Hydriden repräsentiert.

2. Verfahren nach Anspruch 1, **dadurch gekennzeichnet, dass** der Gehalt durch Berechnen einer Fläche H ermittelt wird, die sich aus der Gesamtheit der für die Hydriden repräsentativen Pixelgruppen (21') des Bildes zusammensetzt, wobei die Fläche H mit einer Kalibrationskurve (61) verglichen wird, die von Referenzproben mit bekanntem Wasserstoffgehalt gebildet wird, wobei eine für ein Hydrid repräsentative Pixelgruppe vorbestimmte Messwertoptionen erfüllt, wobei die Kalibrationskurve eine Kurve ist, die für die Fläche H in Abhängigkeit vom Wasserstoffgehalt repräsentativ ist, wobei die Kalibrationskurve von Messpunkten definiert wird, die den in der Fläche H gebildeten Paaren und dem entsprechenden Wasserstoffgehalt der Referenzproben entsprechen, wobei die Berechnung der Fläche H unter Beachtung derselben Messoptionen erfolgt wie für die zu analysierende Probe.

3.  Verfahren nach einem der vorherigen Ansprüche, **dadurch gekennzeichnet, dass** die Metalllegierung eine Zirkoniumlegierung ist.

4.  Verfahren nach einem der vorherigen Ansprüche, **dadurch gekennzeichnet, dass** die Metallegierung eine Schutzhülle für Kernbrennstäbe bildet.

5.  Verfahren nach einem der vorherigen Ansprüche, **dadurch gekennzeichnet, dass** die Fläche H gleich der Summe der Flächen der Pixelgruppen (21') ist, die für Hydride über die Gesamtfläche des Bildes (41) repräsentativ sind.

6.  Verfahren nach einem der vorherigen Ansprüche, **dadurch gekennzeichnet, dass** die Kalibrationskurve (61) den Mindestabstand von der Gesamtheit der Messpunkte der Referenzproben zeigt.

7.  Verfahren nach einem der vorherigen Ansprüche, **dadurch gekennzeichnet, dass** die Länge L des Hydrids durch die Summe der Längen der Zweige seiner skelettierten Darstellung (21') definiert wird.

8.  Verfahren nach einem der Ansprüche 1 bis 6, **dadurch gekennzeichnet, dass** die Länge L des Hydrids durch den Abstand zwischen den zwei entferntesten Punkten seiner skelettierten Darstellung (21') parallel zu einer Achse definiert wird.

9.  Verfahren nach einem der Ansprüche 1 bis 6, **dadurch gekennzeichnet, dass** die Länge L des Hydrids durch den Abstand zwischen den zwei entferntesten Punkten seiner skelettierten Darstellung (21') definiert wird.

10. Verfahren nach einem der vorherigen Ansprüche, **dadurch gekennzeichnet, dass** die Orientierung durch eine mittlere Orientierung $\theta_{moy}$ gemäß der folgenden Relation definiert wird.

$$\theta_{moy} = \frac{\sum_i \theta_i d_i}{\sum_i d_i}$$

wobei i die Reihe des Hydridzweigs, $\theta_1$ die Orientierung der Unterteilung der Reihe i in Bezug auf eine Referenzachse und $d_i$ die Länge der Unterteilung der Reihe i repräsentieren.

11. Verfahren nach einem der Ansprüche 1 bis 9, **dadurch gekennzeichnet, dass** die Orientierung die Orientierung zwischen den zwei entferntesten Punkten des Hydrids ist.

12. Verfahren nach einem der Ansprüche 1 bis 9, **dadurch gekennzeichnet, dass** die Orientierung die Orientierung zwischen den beiden Schnittpunkten der am weitesten entfernten Zweige des Hydrids sind.

13. Verfahren nach einem der vorherigen Ansprüche, **dadurch gekennzeichnet, dass**, da die Nähe p durch den Abstand (81) zwischen der Nähe der nächsten Nachbarhydride definiert wird, wobei dieser Abstand durch die Berechnung des Abstands zwischen jedem Ende eines Hydrids (21') und den Enden der anderen Hydride (21') über einen Winkel zwischen 0° und 180° definiert wird, für jedes Ende das Minimum dieses Abstands gewählt wird, wobei der Wert der Nähe das Minimum der Mindestabstände ist.

14. Verfahren nach Anspruch 13, **dadurch gekennzeichnet, dass** die Berechnung des Abstands zwischen jedem Ende eines Hydrids (21') und den Enden der anderen Hydride (21') über einen Winkel zwischen 0° und 180° einerseits und zwischen 180° und 360° andererseits erfolgt.

15. Verfahren nach einem der vorherigen Ansprüche, **dadurch gekennzeichnet, dass** ein Gefahrenfaktor $D_{moy}$ für die Probe definiert wird, wobei dieser Gefahrenfaktor $D_{moy}$ der Mittelwert der Gefahrenfaktoren der auf dem Bild (41) der Probe vorhandenen Hydride ist.

16. Verfahren nach einem der vorherigen Ansprüche, **dadurch gekennzeichnet, dass** ein maximaler Gefahrenfaktor $D_{max}$ für die Probe definiert wird, wobei dieser Gefahrenfaktor $D_{max}$ dem maximalen Gefahrenfaktor für alle auf dem Bild (41) der Probe vorhandenen Hydride entspricht.

Détourage de l'image — 1

Correction d'éclairage — 2

Passage en niveau de gris — 3

Seuillage ⇒ binarisation — 4

— 5
Squelettisation

Mesures locales — 10

Mesure de la teneur en hydrogène par étalonnage à l'aide d'échantillons de référence — 6

# FIG.1

FIG.2a

FIG.2b

FIG.2c

FIG.2d

FIG.3

FIG.4

Calcul de l'aire H
de l'image  —51

Comparaison de l'aire H calculée
avec une courbe d'étalonnage  —52

FIG.5

FIG.6

FIG.7

FIG.8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **Zhang et al.** Quantification et caractérisation des hydrures de zirconium dans le Zircaloy-4 par analyse d'image. *Journal of Nuclear Materials,* 1992, vol. 195, 17-23